# EUROPEAN PATENT APPLICATION

(11) **EP 4 094 844 A1**
(43) Date of publication of application: **30.11.2022**
(21) Application number: 21175730.7
(22) Date of filing: 25.05.2021
(51) Int. Cl.: B05B 11/04, B65D 83/00, A45D 40/00, A61M 35/00, B65D 47/26

(54) **DISPENSER**

(71) Applicant: MC2 Therapeutics A/S, 2970 Hørsholm (DK)
(72) Inventor: LANGE, Jesper J., 2970 Hørsholm (DK); LARSEN, Jakob Grupe, 3450 Allerød (DK); LUND, Thomas, 3450 Allerød (DK)
(74) Representative: Budde Schou A/S

(57) **Abstract**

The present invention relates to a dispenser (1) for dispensing a pasty or viscous material contained in a collapsible content container (5), said dispenser (1) comprising a container body (2) and a container cap (3). The container body (2) has a side wall with at least an area being elastic deformable, and the container body (2) is adapted for containing the collapsible content container (5). The dispenser (1) also comprises a lid (6) for closing the container body (2) and an outlet device (10). The lid (6) and the outlet device (10) are adapted for being covered by the container cap (3). The container body (2) and lid (6) when engaged forms a closed chamber (16) around the collapsible content container (5), which closed chamber (16) can be pressurized by deforming the deformable area on the container body (2), thereby pressurize the collapsible content container (5) such that content is transported out of the collapsible content container (5) to be dispensed by the dispenser (1). When the collapsible content container (5) is emptied, it may be possible for the user to replace it with a full collapsible content container (5).

## Description

The present invention relates to a dispenser for dispensing a liquid material contained in a collapsible content container.

### BACKGROUND ART

Dispensers have been widely available on the market in a wide variety of designs for years and are primarily used for cosmetics and topical drugs and medical devices for the purpose of dispensing liquid products, such as, for example, creams, lotions, gels and the like.

Dispensers are used more and more for topical applications, especially in the pharmaceutical field, such as for treating skin diseases with creams, ointments and the like.

Compressible dispensers are typically suitable for delivering a liquid product by the action of manual pressure applied to the walls thereof. The compressible dispensers may be of the type where the liquid product contained is not in contact with the air when being dispensed, which helps ensure the stability and preservation of the product.

This type of dispenser is intended for daily use, sometimes even for use multiple times a day. The dispenser is often carried by the user, for example in a pocket or handbag. In these circumstances, accidental seepage should be prevented. Provision must therefore be made for a means of protection, such as a cap. However, this type of protection means provided must not be to the detriment of the aesthetic appeal or use of the product. It is therefore indispensable, on the one hand, for the cap not to be such as to become detached from the body at the slightest pressure and, on the other hand, for the cap not to be attached by means requiring too much force or having too many constraints to remove the cap manually.

Moreover, a compressible dispenser should be easily manipulated and not be bulky. It must be possible for the compressible dispenser to be held in one hand and pressed by the same hand. It should also have the best possible functionalities while preserving the aesthetic characteristics expected in the field of cosmetics.

Another type of dispensers has a substantially rigid reservoir for the liquid product. Such dispenser typically has a pump device to transport the liquid product from the reservoir to the place of application. The pump device typically has a rather complex structure and may include a piston or metallic parts such as a spring. Current dispensers are disposables which causes major environmental issues with plastics and other waste.

However, most dispensers are typical mass-produced products which, on the one hand, depends on a simple structure and easy manufacturability and, on the other hand, it must also be possible to achieve a good visual design, especially in the application in the cosmetics sector. It is naturally difficult to fulfil all these requirements when designing a dispenser.

### DISCLOSURE OF THE INVENTION

An object of the present invention is to provide a dispenser which can be re-used and with a simple and aesthetical design.

A further object is to provide a dispenser which is un-complicated and reliable to use and handle and which is easy to transport by a user.

Moreover, the present invention provides a dispenser which is made from material selected within substantially the same type of material and thereby easier to dispose in a more environmental friendly manner. The dispenser can also be provided with a smooth surface which is easy to clean and improve hygiene.

In a first aspect the present invention relates to a dispenser for dispensing a liquid material contained in a collapsible content container, where the dispenser comprises a container body and a container cap,
the container body being substantially cup-shaped with a bottom and a sidewall and an opening opposite to the bottom, the container body being adapted for containing the collapsible content container,
the opening in the container body being closable by a lid integrated with the collapsible content container, the lid comprises a lid part with a hole adapted for transporting the content from the collapsible content container, and the lid being adapted for engagement with an outlet device,
the lid and the outlet device being adapted for being covered by the container cap, and the outlet device being adapted for attachment to the lid part and comprising a tubular member extending from the lid part to an exit opening in the container cap, where the tubular member can be positioned such that it communicates with the hole in the lid part such that content from the collapsible content container can be transported in the tubular member from the hole in the lid part to the exit opening in the container cap,
wherein the container body and lid when engaged forms a closed chamber around the collapsible content container, wherein the closed chamber can be pressurized, and thereby pressurize the collapsible content container such that content is transported out of the collapsible content container to the exit opening in the container cap via the hole in the lid part and the tubular member.

The terms "liquid", "content", "material" and their plural forms are used interchangeable in this context and used to denote the material contained in the collapsible content container.

The closed chamber may be pressurized by deforming at least one elastic deformable area on the container body. The user may simply squeeze on the container body and deform the elastic deformable area, thereby reducing the volume such that the pressure increases in the closed chamber.

The closed chamber may also be pressurized by activating a pump device or source for compressed gas. The pump device may be an electric operated pump located inside or communicating with the closed chamber. The dispenser can include bottoms or contacts for operating the pump. Alternatively, the pump is operated remotely, e.g. by means of a remote control or a smart phone. The pump can also be a manually operated pump, e.g operated by pushing a bottom one or more times.

It is also possible to pressurize the closed chamber by applying a source for compressed gas, such as a cartridge with nitrogen or dinitrogen oxide (laughter gas). The cartridge can be mounted in a device in the closed chamber and be released from the device, e.g. by activating a bottom or remote control.

The dispenser may comprise re-usable parts and single-use parts, and preferably, the container body, the container cap and the outlet device are re-usable and the collapsible content container and optionally the lid are single-use parts in the dispenser. Thus, in an embodiment, when the collapsible content container is empty, it is possible to replace the empty collapsible content container with a new collapsible content container filled with material to be dispensed.

The lid comprises a lid part which preferably is a protrusion in the central part of the lid on the surface opposite the surface facing the collapsible content container. The lid part may serve as attachment point or attachment device for the container cap to the container body.

The collapsible content container with the material to be dispensed is located in the container body which by means of the lid is sealed thereby forming an air-tight chamber around the collapsible content container which can then be pressurised by e.g. compressing the container body. In use the visible parts of the dispenser are the container body and the container cap.

The dispenser may have any desired cross-section in term of shapes, such as circular, triangular or square, and in a preferred embodiment the dispenser has an elliptical shape. An elliptical shape makes it easy for a user to identify the position of the exit opening in the container cap. The area of an ellipse is normally defined be a major axis and a minor axis, where the major axis is the axis extending in the longest extension of the ellipse and the minor axis is the axis perpendicular to the major axis in the mid-point of the major axis of the ellipse. In case the cross-section is shaped as a circle the major axis and the minor axis would have the same length. However, in the context of this invention the term major axis should be construed as the axis in which the centre of the hole in the lid part is located. The skilled person will be able to transfer this context to other geometric shapes of the cross-section.

Consequently, in an embodiment of the dispenser the lid has a cross-section substantially corresponding to the cross-section of the container body and the cross-section of the container cap, and the lid part is located in a central area of the lid such that the major axis of the lid cross-section divides the lid part in two half parts of the same size and where the hole in the lid part is located off-set in respect of the centre of the major axis of the lid cross-section. In the embodiment lid part is also divide by the minor-axis in substantially two uniform half-parts.

In an embodiment of the dispenser the hole in the lid part is preferably located such that the centre of the cross-section of the hole is located on the major axis of the lid cross-section. The centre of the cross-section of the hole is displaced from the mid-point of the major axis, i.e. the point where the major-axis and the minor axis cross each other. This displacement makes it possible to establish connection between the hole and the outlet device and cut-off the connection by turning the container cap in respect of the container body as it will be explained in more details below.

In an embodiment of the dispenser the tubular member of the outlet device extends in a direction corresponding to the direction of the major axis of the lid cross-section. The tubular member is typically has an "L" shape, and the long part of the "L" extends in a direction parallel with the major axis. The short part of the "L" is perpendicular to this direction and adapted to engage with the hole in the lid part.

As previously mentioned, it is preferred that the container cap can rotate in respect of the container body. The container cap can rotate between a first position and a second position, such that in the first position there is connection between the hole in the lid and the tubular member. This first position in referred to as open mode. In the second position the connection between the hole in the lid and the tubular member is cut off, and this position is referred to as closed mode. The rotation of the container cap in respect of the container body is preferably 180 degrees. In an embodiment the container cap is attached to the lid part and rotates around the lid part which is preferably shaped as a protrusion on the lid. The lid part may include grooves or thread for better attachment between the lid and the container cap.

In an embodiment of the dispenser the container body comprises a valve, preferably a one-way valve, preferably in the bottom. The valve may serve to equalize the pressure in the closed chamber around the collapsible content container, such that the pressure corresponds to the surrounding pressure after an actuation is applied.

The invention also provides an embodiment of the dispenser where the lid or the lid part comprises a one-way valve. This valve may also serve to equalize the pressure in the closed chamber around the collapsible content container and can be considered to be an alternative to the solution where the valve is mounted in the container body. Both solutions establish connection between the closed chamber and the surrounding environment, such that the pressure in the closed chamber can be equalized to correspond to the pressure in the environment surrounding the dispenser. However, the solution with the valve in the lid or the lid part requires that the container cap and the outlet device does not make an airtight attachment to the container body, and preferably the valve should be mounted close to the lid periphery to optimize the access to the closed chamber.

In an embodiment of the dispenser a small temporary vacuum may be created in the closed chamber when the actuation pressure is released on the deformable area on the container body, The small vacuum will affect that some of the material in the outlet channel is sucked back, resulting in a clean cut of the dispensed material flow, and avoid excess material on the outside of the container. The small vacuum may be created by a delay in the one-way valve, such that pressure in the closed chamber is equalised only after a period from the squeezing on the container body has stopped, such that the temporary vacuum appears.

The dispenser may also have a locking or stop function which can be enabled by opening or de-activating the one-way valve placed in the bottom of the closed chamber, which will prevent the activating pressure in the closed chamber from occurring.

In an embodiment the outlet device is integrated with the lid and/or the lid and the collapsible content container. Thus, when the lid and the collapsible content container is replaced in the container body, the outlet device will also be replaced

In an alternative embodiment of the dispenser the outlet device is integrated with the container cap. Thus, the outlet device can be re-used in the dispenser with the container cap.

In an embodiment the collapsible content container optionally with the lid can be recycled and re-used in the dispenser.

The tubular member preferably comprises a valve at the end engaging with the exit opening in the container cap, and preferably the valve is a one-way valve. The one-way valve serves to ensure that the pasty or viscous material can leave the dispenser via the exit opening in the container, but material which have been exposed to the surrounding environment cannot enter into the outlet device. In this manner undesired contamination can be avoided.

In an embodiment of the dispenser the container body, the container cap, the lid and the outlet device is manufactured from the same material, preferably a polymer material such as polyethylene, polypropylene, polyurethane, polystyrene, polyvinylchloride, or a variant of thermoplastic elastomers.

By producing most or all parts of the dispenser within the same type of material recycling of the dispenser becomes easier when disposed.

However, in an embodiment the container body and the container cap are made from metallic material, such as steel or aluminium. Thus, it is possible to provide a long-lasting and durable dispenser, which can be re-used several times.

Preferable the collapsible content container and/or the lid integrated with the collapsible content container is replaceable. Thus, at least the container body and the container cap can be re-used several times and the collapsible content container with the lid can be replaced when empty.

When the dispenser is intended for dispensing a specific product such as a medical cream it is desirable that only a collapsible content container containing the specific product is able to be placed in the container body of the dispenser and that the liquid material matches with the specific text applied on the container body. Thus, the rim of the container body and the lid or spout of the collapsible content container may be adapted with special measures or geometries such as taps or protrusions and recesses or grooves or other physical guiding means which ensure that only a collapsible content container with a specific content can be placed in the dispenser and collapsible content containers containing other products or collapsible content containers from other suppliers will not be able to be placed in the container body and dispensed by the dispenser. Thus, in this embodiment only a collapsible content container where guiding means of the lid and the container body engage can be placed in the container body.
The guiding means can also be of digital character, for instance via a response chip that allow use or not.

Alternative, the lid of the collapsible content container and the container cap can comprise guiding means and be able to engage to allow dispensing of the product in the collapsible content container.

In an embodiment of the dispenser the collapsible content container is a bag, preferably manufactured from a foil. The foil may be a metallic foil, however, the foil may also be made from polymer material. In the latter case the entire dispenser can be made from plastic and rubber.

The liquid material can be pasty or viscous material or have lower viscosity like water. The liquid material can be a cosmetic product or a pharmaceutical product and preferably the material is selected from the group consisting of cream, ointment, oil, lotion, gel, and balsam. The liquid material contained in the collapsible content container can also be a food product such as a dressing, mayonnaise and the like.

In an embodiment the disperser comprises an electronic device, such as an audio or visual alarm, vibration feature for user interaction, a metering device, a score device, a communication device, a display or registration of use including storing of history which can be used for optimisation of individual treatment and future use. The electronic device is preferably battery operated. The alarm may inform a user about the time for applying the material in the dispenser. A metering device may ensure that the correct amount of material is dispensed from the dispenser and a score device may serve to motivate the user to use the dispenser. When the electronic device is a communication device, the device may be able to communicate with another electronic device such as a tablet or a smart phone. The dispenser may also comprise a display which can show information to the user, e.g. if the collapsible content container is empty and how many times collapsible content container has been replaced. Moreover, the dispenser may also include a registration of use including storing of history which can be used to optimise the individual treatment and future use, thereby improving the overall health and well-being of the user. The dispenser may comprise one or more electronic devices.

### DETAILED DESCRIPTION OF THE INVENTION

The invention will now be described in further details with reference to drawings in which:
Figure 1: shows a dispenser according to the invention;
Figure 2: shows a dispenser with collapsible content container;
Figure 3: shows a dispenser with collapsible content container;
Figure 4: shows the dispenser with container cap of;
Figure 5: is a transverse section of the dispenser in open mode;
Figure 6: shows a transverse section with the outlet device in closed mode;
Figure 7: shows rotation of the container cap;
Figure 8: shows the dispenser;
Figure 9: shows the dispenser with the outlet device integrated in the container cap;
Figure 10: shows the dispenser with the outlet device integrated in the container cap.

The figures are only intended to illustrate the principles of the invention and may not be accurate in every detail. Moreover, parts which do not form part of the invention may be omitted. The same reference numbers are used for the same parts.

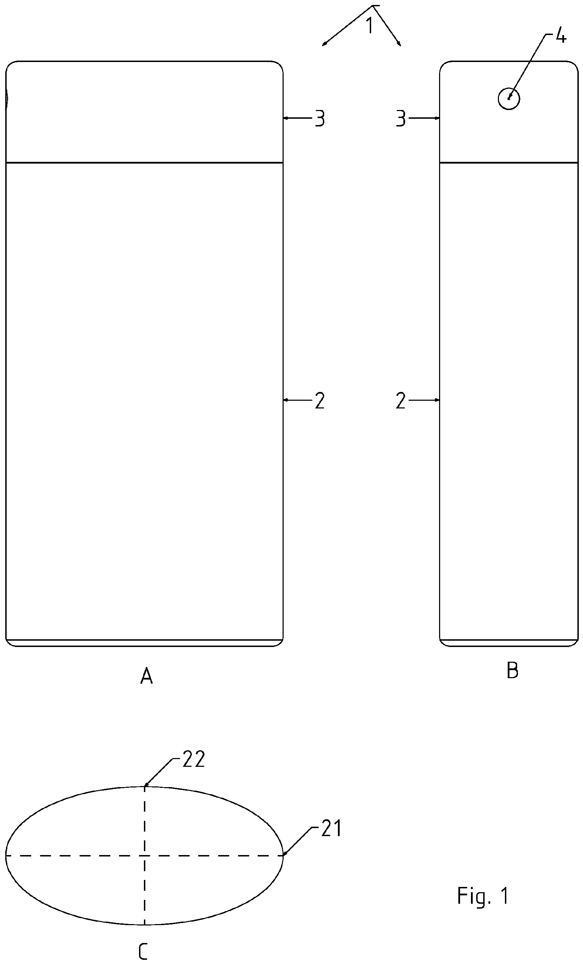

Figure 1 illustrates an embodiment of a dispenser 1 according to the invention. Figure 1 A shows the dispenser 1 from the side with the container body 2 and the container cap 3. In figure 1 B is the dispenser is seen from the front with the container body 2 and the container cap 3. In the front part of the container cap 3 the exit opening 4 can be seen.

Figure 1 C is a top view of the dispenser 1 showing a cross-section shaped as an ellipse. In the figure the major axis 21 and the minor axis 22 are indicated with dotted lines. It is clear that if the cross section was circular the major axis and the minor axis would have the same length.

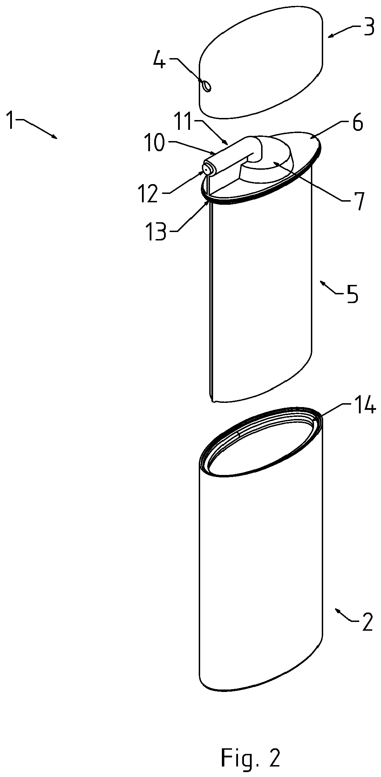

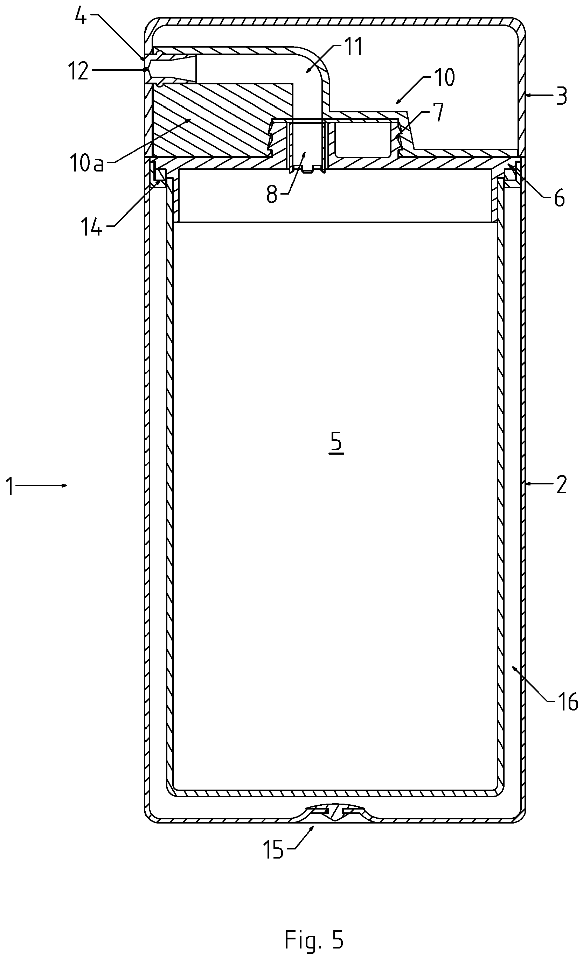

Figure 2 illustrates the dispenser 1 where the container body 2 and the container cap 3 are apart and the collapsible content container 5 with lid 6 can be seen. The lid 6 comprise a lid part 7 in the central portion of the lid 6. However, in this embodiment the lid 6 and the lid part 7 are covered by the outlet device 10 which can be seen more clearly in figure 5.

The exit device 10 comprise a tubular member 11 which at one end is closed with a one-way valve 12. The tubular member 11 and the one-way valve 12 are adapted for engagement with the exit opening 4 in the container cap 3.

The lid 6 further comprises a rim 13 with a packing device to engage with a packing 14 in the container body 2.

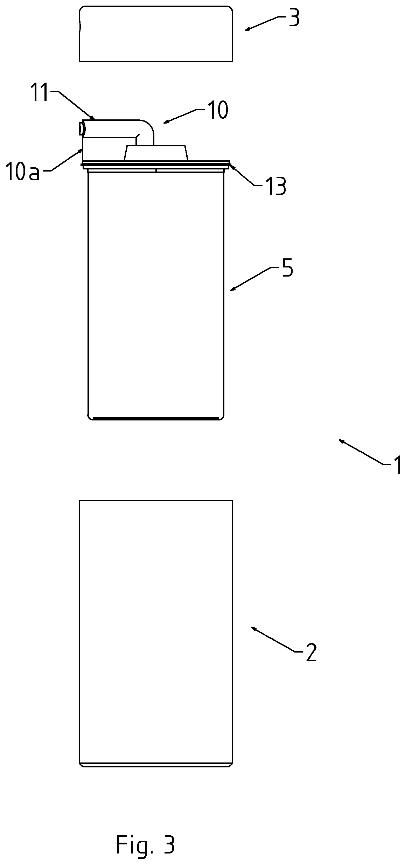

Figure 3 shows the dispenser 1 from a side view, with the container body 2 and container cap 3 apart, and between them the collapsible content container 5 with outlet device 10 and rim 13. As it can be seen the outlet device 10 comprises a support device 10a supporting the tubular member 11 and providing for a more stable function of the dispenser device.

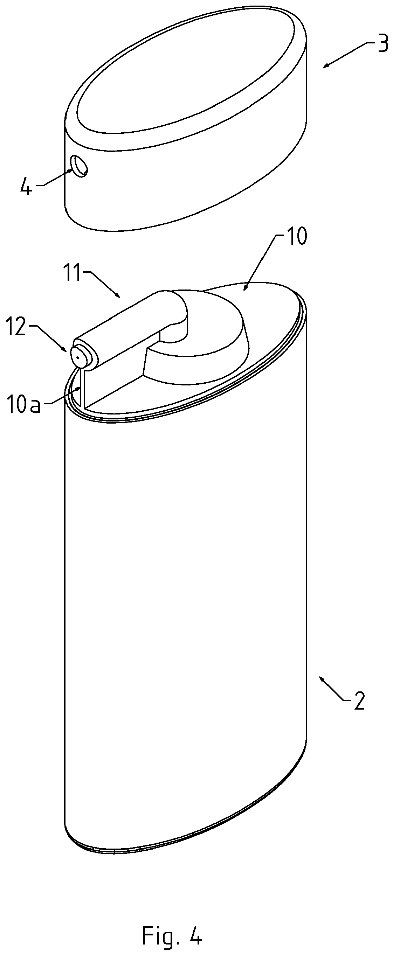

Figure 4 shows the dispenser 1 with container cap 3 dismounted from the container body 2 and the collapsible content container mounted in container body 2. In this embodiment the outlet device 10 is integrated with the collapsible content container and covers the lid.

Figure 5 shows a cross-section of the dispenser 1 in the mode where it is able to dispense material from the collapsible content container 5. The container body 2 and the container body 3 are connected and the container body 2 is closed by the lid 6, which by engagement of the rim 13 and the packing 14 on the container body 2 forms a tight sealing of the container body 6, such that a chamber 16 is formed around the collapsible content container 5.

The container body 2 comprises a one-way valve 15 at the bottom, which blocks during applied pressure, preventing air from leaving the chamber 16, but allows air to flow into the chamber 16 when the user releases the pressure, and the container body returns to the resting mode and shape. In the mode shown in figure 5, the open mode, there is connection between the interior of the collapsible content container 5 and the tubular member 11 in the outlet device 10 via the hole 8 in the lid part 7. The outlet device 10 is attached to the lid part 7 and rotatable around the lid part 7. Both the lid part 7 and the outlet device 10 comprises grooves which improve the attachment between the two parts. In this embodiment the outlet device 10 is integrated with the lid 6 and the collapsible content container 5.

When the wall of the container body 2 is squeezed a higher pressure is established in the chamber 16. This pressure affects the collapsible content container 5 and the pressure squeezes material out of the collapsible content container 5 into the hole 8 and further into the tubular member 11 of the outlet device 10. From the tubular member 11, the material can be dispensed from the dispenser via the one-way valve 12 and the exit opening 4 in the container cap 2. When the container body 3 is no longer squeezed the pressure in the chamber 16 is normalized by air entering the chamber via the one-way valve 15.

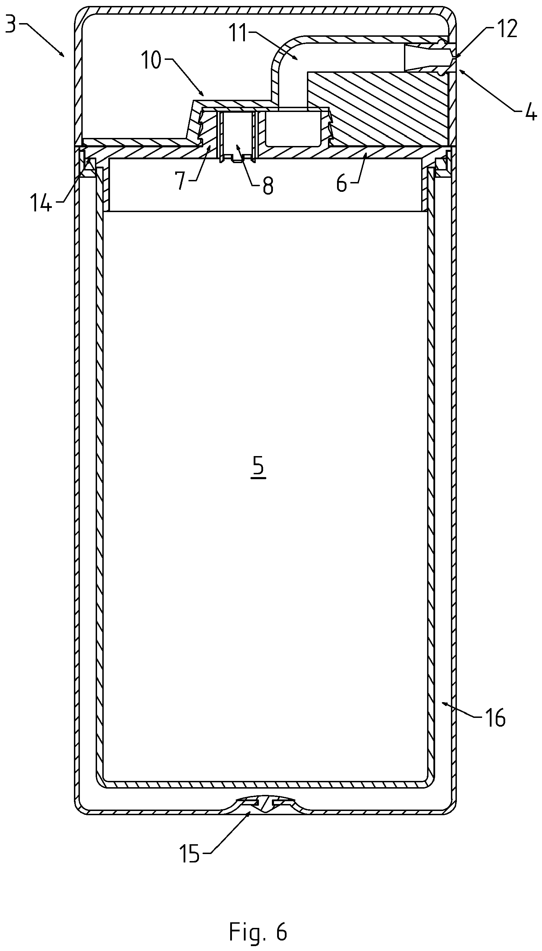

Figure 6 shows the situation where the connection between collapsible content container 5 and the tubular member 11 is disconnected and referred to as the closed mode. This is done by rotating the container cap 3 in respect of the container body 2. The container cap is rotated 180 degrees around the lid part 7 such that the solid part of the lid part 7 blocs the opening in the hole 8 and in the tubular member 11. This is due to the fact that the hole 8 is placed offset in respect of the centre of the major axis, and the tubular member 11 is adapted such that when the dispenser is in the open mode the inlet opening of the tubular member 11 matches with the hole 8 in the lid 7 part as illustrated in figure 5.

As it appears from figure 5 and 6 that the collapsible content container 5 is attached to the lid 6. The attachment is preferably by welding.

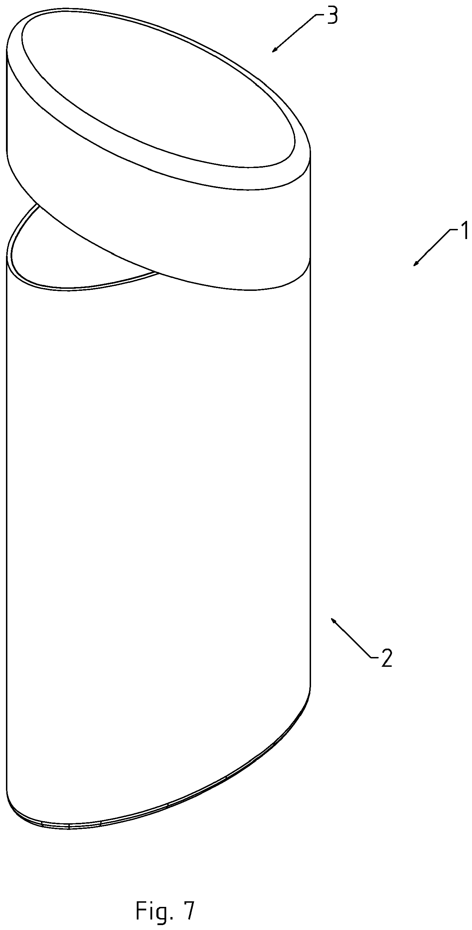

Figure 7 illustrates how the container cap 3 is rotated in respect of the container body 2. By rotating the container cap 3 it is possible to activate or deactivate the dispenser to be in the open mode or closed mode, respectively, as explained above.

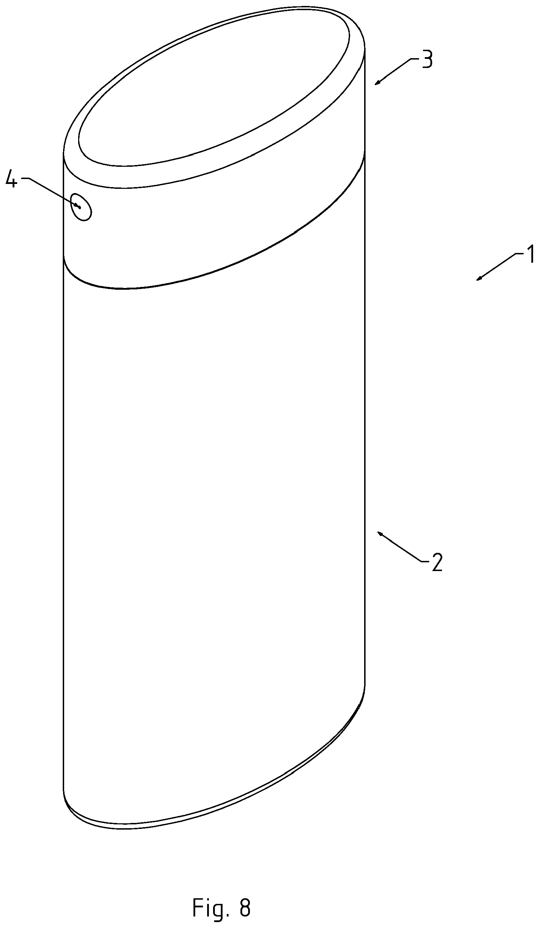

Figure 8 shows the dispenser 1 in activated mode where the container body 2 carrying the collapsible content container is in the position in respect of the container cap 3 such that material can be dispensed via the exit opening 4.

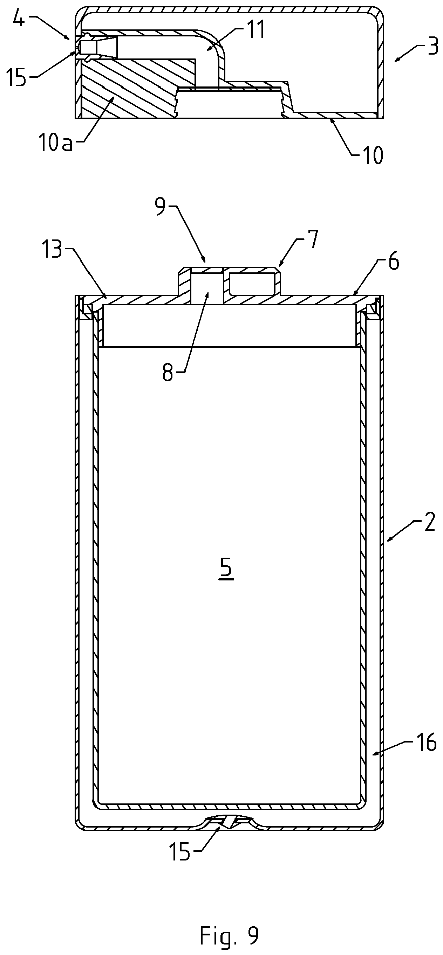

Figure 9 shows an embodiment where the outlet device 10 is integrated in the container cap 3 such that the outlet device 10 engage with the lid 6 and the lid part 7 on the container body 2 when the container cap 3 is mounted on the container body 2.

The collapsible content container 5 with the lid 6 is replaceable mounted in the container body 2 and the lid part 7 can attach to the outlet device 10. As the collapsible content container 5 with the lid 6 is a replaceable part and stored separately before it is mounted in the container body 2, the hole 8 in the lid part 7 is closed with a closure 9 which can be broken or removed when the container cap 3 engages with the container body 2 such that communication is established between the hole 8 and the inlet of the tubular member 11 for dispensing material from the collapsible content container 5 via the hole 8, the tubular member 11 and the one way valve 15 through the exit opening 4 in the container cap 3.

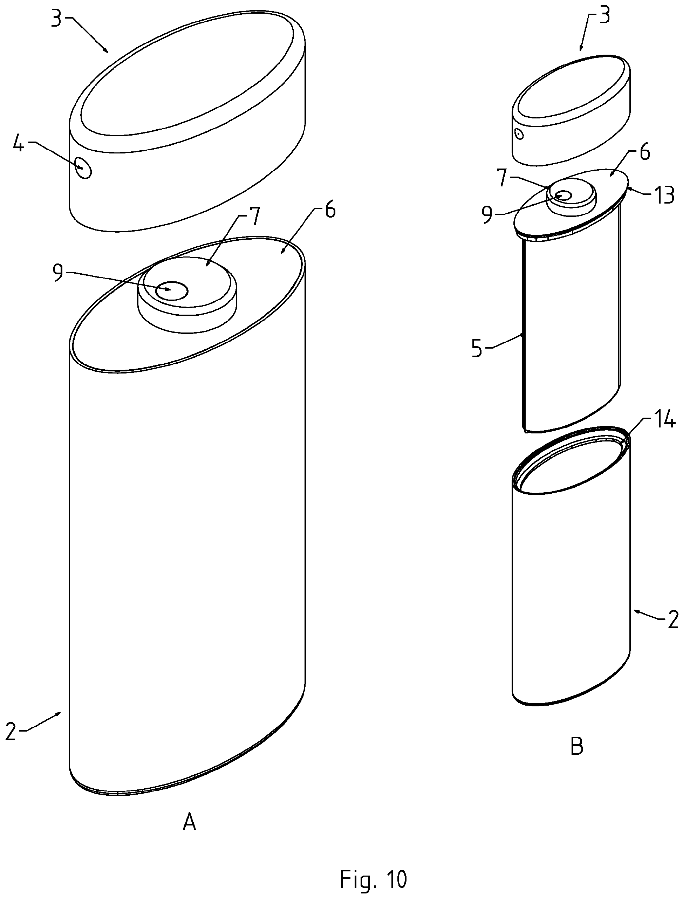

Figure 10 shows the dispenser 1 with the outlet device integrated in the container cap 3.

Figure 10 A shows the dispenser 1 with the collapsible content container 5 including the lid 6 and the lid part 7 mounted in the container body 2. When the container cap 3 is mounted on the lid 6 and lid part 7 in the container body 2 the closure 9 is broken and the material in the collapsible content container can be dispensed. The closure 9 serves to preserve the material in the collapsible content container 5 until use in the dispenser. When the dispenser 1 is assembled the dispensing function can be temporary cut off by turning the container cap 3 in respect of the container body 2 between the open mode and the closed mode as explained above.

Figure 10 B shows the dispenser where the collapsible content container 5 with lid 6 and lid part 7 is not mounted in the container body 2. This also illustrates the principles how the collapsible content container 5 can be mounted in the container body 2 by guiding the collapsible content container 5 into the container body 2 and press the rim 13 with packing towards the packing 14 in the container body 2 such that a tight connection is established.

Although the figures illustrate the dispense with ellipse cross-section it will be clear that the dispenser may have other shapes or cross-sections such as circular or square. It is also clear that the cross-section need not being constant from the top to the bottom of the dispenser but may vary. The cross section may e.g. be smaller at the top and the bottom of the dispenser, than at the middle portion of the dispenser. The dispenser may also comprise more closures and packings than shown in the above figures. The surface of the dispenser may also be provided with text and/or logo and trademark.

### List of reference numbers:

- 1: dispenser
- 2: container body
- 3: container cap
- 4: exit opening
- 5: collapsible content container
- 6: lid
- 7: lid part
- 8: hole
- 9: closure
- 10: outlet device
- 10a: support member
- 11: tubular member
- 12: one-way valve
- 13: rim with packing
- 14: packing
- 15: valve
- 16: closed chamber
- 21: major axis
- 22: minor axis

## Claims

1. A dispenser for dispensing a liquid material contained in a collapsible content container, said dispenser comprising a container body and a container cap,
said container body being substantially cup-shaped with a bottom and a sidewall and an opening opposite to the bottom, said container body being adapted for containing said collapsible content container,
said opening in the container body being closable by a lid integrated with the collapsible content container, said lid comprises a lid part with a hole adapted for transporting the content from the collapsible content container, said lid being adapted for engagement with an outlet device,
said lid and said outlet device being adapted for being covered by the container cap,
said outlet device being adapted for attachment to the lid part and comprising a tubular member extending from the lid part to an exit opening in the container cap, where the tubular member can be positioned such that it communicates with the hole in the lid part such that content from the collapsible content container can be transported in the tubular member from the hole in the lid part to the exit opening in the container cap,
wherein said container body and lid when engaged forms a closed chamber around the collapsible content container, wherein said closed chamber can be pressurized, and thereby pressurize the collapsible content container such that content is transported out of the collapsible content container to the exit opening in the container cap via the hole in the lid part and the tubular member.

2. A dispenser according to claim 1, wherein the closed chamber is pressurised by deforming at least one elastic deformable area on the container body or by activating a pump device or source for compressed gas.

3. A dispenser according to claim 1 or 2, wherein the lid has a cross-section substantially corresponding to the cross-section of the container body and the cross-section of the container cap, and the lid part is located in a central area of the lid such that the major axis of the lid cross-section divides the lid part in two half parts of the same size and where the hole in the lid part is located off-set in respect of the centre of the major axis of the lid cross-section.

4. A dispenser according to any one of the preceding claims, wherein the hole in the lid part is located such that the centre of the cross-section of the hole is on the major axis of the lid cross-section.

5. A dispenser according to any one of the preceding claims, wherein the tubular member of the outlet device extends in a direction corresponding to the direction of the major axis of the lid cross-section.

6. A dispenser according to any one of the preceding claims, wherein the container cap can rotate in respect of the container body between a first position and a second position, such that in the first position there is connection between the hole in the lid and the tubular member and in the second position the connection between the hole in the lid and the tubular member is cut off.

7. A dispenser according to any one of the preceding claims, wherein the container body comprises a one-way valve, preferably in the bottom.

8. A dispenser according to any one of the preceding claims 1-6, wherein the lid or the lid part comprises a one-way valve

9. A dispenser according to any one of the preceding claims, wherein outlet device is integrated with the lid and/or the lid and the collapsible content container

10. A dispenser according to any one of the preceding claims, wherein the outlet device is integrated with the container cap.

11. A dispenser according to any one of the preceding claims, wherein the tubular member comprises a valve at the end engaging with the exit opening in the container cap, preferably a one-way valve.

12. A dispenser according to any one of the preceding claims, wherein the container body and the lid of the collapsible content container are adapted to engage by means of taps and recesses or other guiding means such that only a collapsible content container adapted with special taps and/or recesses will fit in the container body or guiding means including a digital character, for instance via a response chip that allow use or not.

13. A dispenser according to any one of the preceding claims, wherein the container body, the container cap, the lid and the outlet device is manufactured from the same material, preferably a polymer material, and preferably the collapsible content container is a bag, preferably manufactured from a foil and preferably collapsible content container or the lid integrated with the collapsible content container is replaceable.

14. A dispenser according to any one of the preceding claims, wherein the liquid material is selected from the group consisting of cream, lotion, liquid oil, gel, ointment, and balsam.

15. A dispenser according to any one of the preceding claims, wherein the disperser comprises an electronic device, such as an audio or visual alarm, vibration feature for user interaction, a metering device, or a score device or a communication device or a display or registration of use including storing of history which can be used for optimisation of individual treatment and future use.
